Europäisches Patentamt

European Patent Office

Office européen des brevets

(19)

(11) Publication number: **0 197 633**
**A1**

(12) **EUROPEAN PATENT APPLICATION**

(21) Application number: **86300973.4**

(22) Date of filing: **12.02.86**

(51) Int. Cl.⁴: **C 07 C 89/00**
**C 07 C 91/44**

(30) Priority: **19.03.85 JP 55010/85**

(43) Date of publication of application:
**15.10.86 Bulletin 86/42**

(84) Designated Contracting States:
**DE FR GB IT**

(71) Applicant: **SUMITOMO CHEMICAL COMPANY, LIMITED**
**15 Kitahama 5-chome Higashi-ku**
**Osaka-shi Osaka 541(JP)**

(72) Inventor: **Harada, Haruhisa**
**7-23-5 Aobadai**
**Ichihara-shi Chiba(JP)**

(72) Inventor: **Maki, Hiroshi**
**1353-4, Shiizu**
**Ichihara-shi Chiba(JP)**

(72) Inventor: **Sasaki, Shigeru**
**1-9, Yushudai Nishi**
**Ichihara-shi Chiba(JP)**

(74) Representative: **Diamond, Bryan Clive et al,**
**Gee & Co. Chancery House Chancery Lane**
**London WC2A 1QU(GB)**

(54) Method for the production of m-aminophenol.

(57) A method for the production of m-aminophenol comprises non-catalytically reacting resorcinol and ammonia at a temperature between 180 and 300°C and at a pressure of from 25 to 80 kg/cm² (gauge) in the presence of water.

The pressure can be maintained by supply of inert gas.

The product can be isolated by distillation.

The elevated pressure gives good conversion of the resorcinol and selectivity for the product m-aminophenol without substantially increasing the molar ratio of ammonia to resorcinol.

EP 0 197 633 A1

Croydon Printing Company Ltd.

# PRODUCTION OF m-AMINOPHENOL FROM RESORCINOL

The present invention relates to a method for non-catalytic production of m-aminophenol which is industrially very important not only as an intermediate for the preparation of medicines, agricultural chemicals, azo dyes, antioxidants, and photographic developers but also as a starting material for the manufacture of polyimide- or polyamide-based heat resistant resins.

Hitherto, m-aminophenol was produced by melting metanilic acid together with caustic soda, hydroxylating m-phenylenediamine, or reacting resorcinol and ammonia in an aqueous medium in the presence of a catalyst. As the catalyst, there were used phosphoric acid or ammonium phosphate (Japanese Patent Application (OPI) No. 121728/1978); copper halides, nickel halides, or cobalt halides (Japanese Patent Application (OPI) No. 42829/1977); phosphoric acid salts of antimony, vanadium, or iron (Japanese Patent Application (OPI) No. 4338/1980); and molybdenum compounds (Japanese Patent Application (OPI) No. 108841/1980). (The term "OPI" as used herein refers to a published unexamined Japanese patent application .) Whichever type of catalyst is used, the reaction be carried out in an aqueous medium in the

- 2 -

0197633

presence of a large amount (up to the stoichiometric amount) of the catalyst. Therefore, the product m-aminophenol was isolated by precipitation upon cooling the aqueous solution which had been adjusted neutral, extraction from an aqueous phase, or by distillation. However, in these methods, substantial amounts of salts were precipitated, leading to heavy loads for waste liquor treatment.

This problem was also encountered in the partial hydroxylation of m-phenylenediamine and the melting of metanilic acid together with caustic soda.

As methods for eliminating this problem, Japanese Patent Application (OPI) No. 28429/1973 proposed a method in which the reaction of resorcinol and ammonia is carried out by introducing ammonia gas into molten resorcinol at atmospheric pressure in the absence of both a catalyst and a solvent. This proposed method is a "non-catalytic" method, which is free from the problem involved in the above-described "catalytic" methods. However, this non-catalytic method had a problem that the reaction rate is low, i.e., since ammonia gas is introduced at atmospheric pressure, nonetheless ammonia is introduced in an amount of from 3 to 10 molar times that of resorcinol; the conversion of resorcinol is at most about 25 to 55%. In addition, the selectivity for m-aminophenol is not always high as about from 85 to 77% with respect to resorcinol.

- 3 -

0197633

We have sought a non-catalytic method which is free from the above-described problems, and have found that when the reaction components are reacted under elevated pressure in the presence of water, both the conversion of resorcinol and the selectivity for m-aminophenol can be increased without substantially increasing the molar ratio of ammonia to resorcinol.

The present invention thus provides a method for the production of m-aminophenol, comprising non-catalytically reacting resorcinol and ammonia at a temperature between 180 and 300°C and at a pressure of from 25 to 80 kg/cm² (gauge) in the presence of water.

If the pressure of the reaction system is less than 25 kg/cm² (gauge), no appropriate reaction rate is attained, and low selectivity for m-aminophenol will result. On the other hand, if the reaction is carried out at a pressure exceeding 80 kg/cm² (gauge), the results are no better than when the reaction is performed in the pressure range of from 25 to

80 kg/cm$^2$ (gauge), and no significant advantage is attained by increasing the pressure to higher than 80 kg/cm$^2$ (gauge). The pressure range for the reaction system, i.e., from 25 to 80 kg/cm$^2$ (gauge), can be maintained either by controlling the reaction temperature to be within the range of from 180 to 300°C, or by adjusting the concentration of ammonia in an aqueous solution with the molar ratio of ammonia to resorcinol being held at 1.0/1 or more. If desired, the pressure of the reaction system can be maintained by supplying a gas which is inert to the reaction, such as nitrogen, argon, or helium.

Subsequent to completion of the reaction, the product m-aminophenol can be isolated by distillation; the unreacted resorcinol and the product m-aminophenol are eluted together and can be separated from 3,3'-dihydroxydiphenylamine which is a condensate of resorcinol and m-aminophenol and other condensation products.

The separation of the eluted resorcinol and m-aminophenol can be carried out by recrystallization from water or by liquid-liquid extraction with a water-ether solvent system.

The following examples are provided for the purpose of further illustrating the present invention.

0197633

## EXAMPLE 1

A 500-ml SUS autoclave was charged with 220.2 g (2.0 moles) of resorcinol and 85.0 g (2.5 moles) of a 50% aqueous ammonia solution (which had been prepared from liquid ammonia), and reaction was conducted at 230°C for 2 hours while maintaining the pressure in the reaction system at from 32 to 36 $kg/cm^2$ (gauge). After completion of the reaction, the reaction mixture was analyzed by liquid chromatography. As the result, the conversion of resorcinol and the selectivity for m-aminophenol were 60% and 85%, respectively.

## COMPARATIVE EXAMPLE 1

A 500-ml SUS autoclave was charged with 165.2 g (1.5 moles) of resorcinol and 127.5 g (1.88 moles) of a 25% aqueous ammonia solution, and reaction was conducted at 180°C for 7 hours while maintaining the pressure in the reaction system at 23 $kg/cm^2$ (gauge). After completion of the reaction, the reaction mixture was analyzed to show the reaction result that the conversion of resorcinol and the selectivity for m-aminophenol were 21% and 62%, respectively.

## EXAMPLE 2

A 500-ml SUS autoclave was charged with resorcinol and a 25% aqueous ammonia solution in the same amounts as used in Comparative Example 1, and reaction was conducted at 180°C for 6 hours while supplying nitrogen gas to adjust the pressure in the reaction system at 36 kg/cm$^2$ (gauge). After completion of the reaction, the reaction mixture was analyzed to show the reaction result that the conversion of resorcinol and the selectivity for m-aminophenol were 43% and 83%, respectively.

## COMPARATIVE EXAMPLE 2

In a melt of 2 moles of resorcinol at 160°C, a total of about 10 moles of ammonia gas was introduced at atmospheric pressure over about 6 hours. After completion of the reaction, the reaction mixture was analyzed to show the reaction result that the conversion of resorcinol and the selectivity for m-aminophenol were 23.8% and 81.0%, respectively.

## EXAMPLE 3

A 500-ml SUS autoclave was charged with 192.7 g (1.75 moles) of resorcinol and 93.0 g (2.19 moles) of a 40% aqueous ammonia solution (which had been prepared from liquid ammonia), and reaction was conducted at 250°C for 2 hours while maintaining the pressure in the reaction system at from 38 to 42 kg/cm$^2$ (gauge). After completion of the reaction, the reaction mixture was analyzed to show the reaction result that the conversion of resorcinol and the selectivity for m-aminophenol were 65% and 84%, respectively.

## COMPARATIVE EXAMPLE 3

In a melt of 10 moles of resorcinol at 160°C, a total of about 60 moles of ammonia gas was introduced at atmospheric pressure over about 15 hours. After completion of the reaction, the reaction mixture was analyzed to show the reaction result that the conversion of resorcinol and the selectivity for m-aminophenol were 45.0% and 80%, respectively.

### EXAMPLE 4

A 500-ml SUS autoclave was charged with 220.2 g (2.0 moles) of resorcinol and 70.8 g (2.5 moles) of a 60% aqueous ammonia solution (which had been prepared from liquid ammonia), and reaction was conducted at 230°C for 0.5 hour while maintaining the pressure in the reaction system at from 32 to 36 kg/cm$^2$ (gauge). After completion of the reaction, the reaction mixture was analyzed to show the reaction result that the conversion of resorcinol and the selectivity for m-aminophenol were 37% and 90%, respectively.

CLAIMS:

1. A method for the production of m-aminophenol, comprising non-catalytically reacting resorcinol and ammonia at a temperature between 180 and 300°C and at a pressure of from 25 to 80 $kg/cm^2$ (gauge) in the presence of water.

2. A method as claimed in Claim 1, wherein the molar ratio of ammonia to resorcinol is 1.0:1 or more.

3. A method as claimed in Claim 1 or 2, wherein an inert gas is supplied to maintain said pressure.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | DE-C- 260 234 (E. MERCK)<br>* Example 2 *<br><br>--- | 1-3 | C 07 C 91/14 |
| Y | "METHODEN DER ORGANISCHEN CHEMIE", (HOUBEN-WEYL), 4th edition, vol. XI/1: "Stickstoffverbindungen II", 1957, pages 163-164, Georg Thieme Verlag, Stuttgart, DE.<br>* Page 163, paragraph 3 *<br><br>--- | 1-3 | |
| X | GB-A-1 397 098 (HOECHST-AG)<br>* Page 1, lines 74-82 *<br><br>--- | 1-3 | |
| D,A | CHEMICAL ABSTRACTS, vol. 87, no. 17, 24th October 1977, page 672, abstract no. 134555q, Columbus, Ohio, US; & JP - A - 77 42 829 (ASAHI CHEMICAL INDUSTRY CO., LTD.) 04-04-1977<br>* Abstract *<br><br>----- | 1-3 | **TECHNICAL FIELDS SEARCHED (Int. Cl.4)**<br><br>C 07 C 91/00 |

The present search report has been drawn up for all claims

| Place of search | Date of completion of the search | Examiner |
|---|---|---|
| THE HAGUE | 24-07-1986 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82